(19) Europäisches Patentamt
European Patent Office
Office européen des brevets

(11) EP 0 889 883 B1

(12) # EUROPÄISCHE PATENTSCHRIFT

(45) Veröffentlichungstag und Bekanntmachung des Hinweises auf die Patenterteilung:
**13.03.2002 Patentblatt 2002/11**

(21) Anmeldenummer: **97914214.8**

(22) Anmeldetag: **10.03.1997**

(51) Int Cl.[7]: **C07D 251/70**, D06M 13/00

(86) Internationale Anmeldenummer:
**PCT/EP97/01209**

(87) Internationale Veröffentlichungsnummer:
**WO 97/35848 (02.10.1997 Gazette 1997/42)**

(54) **TRIAZINDERIVATE ALS FIXIERMITTEL BEIM FÄRBEN UND ALS VERNETZER**

TRIAZINE DERIVATIVES AS FIXERS IN COLOURING AND AS CROSS-LINKING AGENTS

DERIVES DE LA TRIAZINE UTILISES COMME FIXATEURS EN TEINTURE ET COMME AGENTS DE RETICULATION

(84) Benannte Vertragsstaaten:
**AT CH DE FR GB LI**

(30) Priorität: **25.03.1996 DE 19611668**

(43) Veröffentlichungstag der Anmeldung:
**13.01.1999 Patentblatt 1999/02**

(73) Patentinhaber: **DyStar Textilfarben GmbH & Co. Deutschland KG**
**65926 Frankfurt am Main (DE)**

(72) Erfinder:
- **PATSCH, Manfred**
  **D-67157 Wachenheim (DE)**
- **KRALLMANN, Reinhold**
  **D-67273 Weisenheim (DE)**
- **REUTHER, Wolfgang**
  **D-69118 Heidelberg (DE)**
- **GRÖSSER, Thomas**
  **D-67067 Ludwigshafen (DE)**

(56) Entgegenhaltungen:
**EP-A- 0 175 225**
**EP-A- 0 682 019**
**WO-A-94/29282**
**DE-A- 4 320 447**
**DE-A- 4 417 719**
**US-A- 3 278 253**
**US-A- 3 400 121**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 889 883 B1

## Beschreibung

Die vorliegende Erfindung betrifft neue Triazinderivate der Formel I

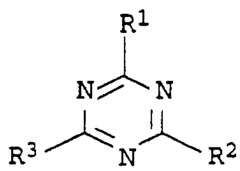

(I),

in der die Variablen die folgende Bedeutung haben:

$R^1$ ist ein Rest der Formel $NL^1L^2$ oder $OL^1$;

$R^2$ ist ein Rest der Formel $NL^1L^2$ oder $OL^1$ oder Halogen;

$R^3$ ist ein Rest der Formel $NL^1L^2$ oder $OL^1$, $C_1$-$C_4$-Alkyl oder Phenyl, das ein- oder zweifach durch Hydroxysulfonyl, Chlor, Nitro, $C_1$-$C_4$-Alkyl, $C_1$-$C_4$-Alkoxy, Cyano, Carboxyl, Acetylamino, Hydroxysulfonylmethyl oder einen Rest der Formel

$$CH_2\text{-}SO_2\text{-}Y,\ SO_2\text{-}Y,\ NL^1\text{-}CO\text{-}Y \text{ oder } NL^1\text{-}CO\text{-}NL^1\text{-}Z\text{-}SO_2\text{-}Y$$

substituiert sein kann;

$L^1$ ist Wasserstoff oder $C_1$-$C_{10}$-Alkyl, das mit einem Rest der Formel -$SO_2$-Y substituiert sein kann und gegebenenfalls durch jeweils 1 bis 3 Sauerstoffatome in Etherfunktion oder nicht benachbarte Imino- oder $C_1$-$C_4$-Alkyliminogruppen unterbrochen ist;

$L^2$ ist ein Rest $L^1$ oder Phenyl, das ein- oder zweifach mit Hydroxysulfonyl, Chlor, Nitro, $C_1$-$C_4$-Alkyl, $C_1$-$C_4$-Alkoxy, Cyano, Carboxyl, Acetylamino, Hydroxysulfonylmethyl oder einem Rest der Formel

$$CH_2\text{-}SO_2\text{-}Y,\ SO_2\text{-}Y,\ NL^1\text{-}CO\text{-}Y,\ CO\text{-}NL^1\text{-}Z\text{-}SO_2\text{-}Y \text{ oder } NL^1\text{-}CO\text{-}NL^1\text{-}Z\text{-}SO_2\text{-}Y$$

substituiert sein kann,
oder $L^1$ und $L^2$ sind, zusammen mit dem sie verbindenden Stickstoffatom, Pyrrolidinyl, Piperidinyl, Morpholinyl, Piperazinyl oder N-($C_1$-$C_4$-Alkyl)piperazinyl;

Z ist $C_2$-$C_6$-Alkylen, das gegebenenfalls durch Hydroxy, Chlor, Cyano, Carboxyl, $C_1$-$C_4$-Alkoxycarbonyl, $C_1$-$C_4$-Alkanoyloxy oder Sulfato substituiert ist und durch jeweils 1 oder 2 Sauerstoffatome in Etherfunktion oder nicht benachbarte Imino-oder $C_1$-$C_4$-Alkyliminogruppen unterbrochen sein kann;

Y ist Vinyl oder ein Rest der Formel $C_2H_4$-Q, worin Q für eine unter alkalischen Reaktionsbedingungen abspaltbare Gruppe steht;

mit der Maßgabe, daß im Rest $R^1$ $L^1$ $C_1$-$C_{10}$-Alkyl, das mit einem Rest der Formel -$SO_2$-Y substituiert ist und gegebenenfalls durch jeweils 1 bis 3 Sauerstoffatome in Etherfunktion oder nicht benachbarte Imino- oder $C_1$-$C_4$-Alkyliminogruppen unterbrochen ist, bedeutet,
ihre Verwendung als Fixierhilfsmittel beim Färben von Hydroxygruppen oder Stickstoffatome aufweisenden organischen Substraten mittels anionischer Farbstoffe sowie als Vernetzer für Regeneratcellulose oder cellulosehaltige Materialien.

Aus K. Venkataraman "The Chemistry of Synthetic Dyes", Band 6, Seiten 201 bis 208, Academic Press, New York, London, 1972, sind Fixierhilfsmittel für das Färben mit anionischen Farbstoffen bekannt.

Es hat sich jedoch gezeigt, daß die dort beschriebenen Verbindungen noch nicht alle Erwartungen erfüllen.

Aufgabe der vorliegenden Erfindung war es daher, neue Triazinderivate bereitzustellen, die in vorteilhafter

Weise zur Anwendung als Fixierhilfsmittel beim Färben von Hydroxygruppen oder Stickstoffatome aufweisenden organischen Substraten mittels anionischer Farbstoffe geeignet sind. Weiterhin sollten sich die neuen Triazinderivate auch als Vernetzer von Regeneratcellulose eignen.

Demgemäß wurden die eingangs näher bezeichneten Triazinderivate der Formel I gefunden.

Wenn die neuen Triazinderivate der Formel I Hydroxysulfonyl- oder Carboxylgruppen aufweisen, so werden selbstverständlich auch deren Salze von den Patentansprüchen umfaßt.

Geeignete Kationen leiten sich von Metall- oder Ammoniumionen ab. Metallionen sind insbesondere die Lithium-, Natrium- oder Kaliumionen. Unter Ammoniumionen im erfindungsgemäßen Sinne sind unsubstituierte oder substituierte Ammoniumkationen zu verstehen. Substituierte Ammoniumkationen sind z.B. Monoalkyl-, Dialkyl-, Trialkyl-, Tetraalkyl- oder Benzyltrialkylammoniumkationen oder solche Kationen, die sich von stickstoffhaltigen fünf- oder sechsgliedrigen gesättigten Heterocyclen ableiten, wie Pyrrolidinium-, Piperidinium-, Morpholinium- oder Piperaziniumkationen oder deren N-monoalkyl- oder N,N-dialkylsubstituierte Produkte. Unter Alkyl ist dabei im allgemeinen geradkettiges oder verzweigtes $C_1$-$C_{20}$-Alkyl zu verstehen, das durch 1 oder 2 Hydroxygruppen substituiert und/oder durch 1 bis 4 Sauerstoffatome in Etherfunktion unterbrochen sein kann.

Alle in der vorliegenden Anmeldung auftretenden Alkyl- und Alkylenreste können sowohl geradkettig als auch verzweigt sein.

Wenn in der vorliegenden Anmeldung substituierte Alkyl- oder Alkylengruppen auftreten, so weisen sie in der Regel 1 oder 2 Substituenten auf.

Im Rahmen der vorliegenden Erfindung steht Halogen für Fluor oder Chlor.

Der Rest Q steht für eine unter alkalischen Reaktionsbedingungen abspaltbare Gruppe. Solche Gruppen sind z.B. Chlor, Brom, $C_1$-$C_4$-Alkylsulfonyl, Phenylsulfonyl, $OSO_3H$, $SSO_3H$, $OP(O)(OH)_2$, $C_1$-$C_4$-Alkylsulfonyloxy, Phenylsulfonyloxy, $C_1$-$C_4$-Alkanoyloxy, $C_1$-$C_4$-Dialkylamino oder ein Rest der Formel

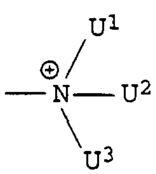 $An^{\ominus}$, 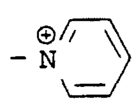 $An^{\ominus}$, 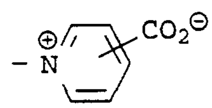 oder 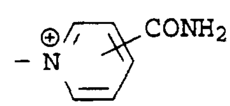 $An^{\ominus}$,

wobei $U^1$, $U^2$ und $U^3$ unabhängig voneinander jeweils die Bedeutung von $C_1$-$C_4$-Alkyl oder Benzyl und $An^{\ominus}$ jeweils die Bedeutung eines Äquivalents eines Anions besitzen. Als Anionen können dabei z.B. Fluorid, Chlorid, Bromid, Iodid, Mono-, Di- oder Trichloracetat, Methansulfonat, Benzolsulfonat oder 2- oder 4-Methylbenzolsulfonat in Betracht kommen.

Wenn in den obengenannten Formeln Alkyl- oder Alkylenreste auftreten, die durch mehrere Sauerstoffatome in Etherfunktion oder Imino- oder $C_1$-$C_4$-Alkyliminogruppen unterbrochen sind, so sind solche Alkyl- oder Alkylenreste bevorzugt, die durch 1 oder 2 Sauerstoffatome in Etherfunktion oder Imino- oder $C_1$-$C_4$-Alkyliminogruppen unterbrochen sind.

Reste $L^1$, $L^2$ und $R^3$ sind z.B. Methyl, Ethyl, Propyl, Isopropyl, Butyl, Isobutyl, sec-Butyl oder tert-Butyl.

Reste $L^1$ und $L^2$ sind weiterhin z.B. Pentyl, Isopentyl, Neopentyl, tert-Pentyl, Hexyl, 2-Methylpentyl, Heptyl, Octyl, 2-Ethylhexyl, Isooctyl, Nonyl, Isononyl, Decyl, Isodecyl. [Die Bezeichnungen Isooctyl, Isononyl und Isodecyl sind Trivialbezeichnungen und stammen von den nach der Oxosynthese erhaltenen Alkoholen (vgl. dazu Ullmann's Encyclopedia of Industrial Chemistry, 5th Edition, Vol. AI, Seiten 290 bis 293, sowie Vol. A 10, Seiten 284 und 285).], 2-Methoxyethyl, 2-Ethoxyethyl, 2-Propoxyethyl, 2-Butoxyethyl, 2- oder 3-Methoxypropyl, 2- oder 3-Ethoxypropyl, 2- oder 3-Propoxypropyl, 2- oder 4-Methoxybutyl, 2- oder 4-Ethoxybutyl, 3,6-Dioxaheptyl, 3,6-Dioxaoctyl, 3,7-Dioxaoctyl, 4,7-Dioxaoctyl, 2- oder 3-Butoxypropyl, 2- oder 4-Butoxybutyl, 4,8-Dioxanonyl, 3,7-Dioxanonyl, 4,7-Dioxanonyl, 4,8-Dioxadecyl, 3,6,9-Trioxadecyl, 3,6,9-Trioxaundecyl, 2-Methylaminoethyl, 2-Dimethylaminoethyl, 2-Ethylaminoethyl, 2-Diethylaminoethyl, 2- oder 3-Methylaminopropyl, 2- oder 3-Dimethylaminopropyl, 2- oder 3-Ethylaminopropyl, 2- oder 3-Diethylaminopropyl, 2- oder 4-Methylaminobutyl, 2- oder 4-Dimethylaminobutyl, 2- oder 4-Ethylaminobutyl, 2- oder 4-Diethylaminobutyl oder Reste der Formel $C_2H_4$-$SO_2$-Y, $C_3H_6$-$SO_2$-Y, $C_4H_8$-$SO_2$-Y, $C_2H_4OC_2H_4$-$SO_2$-Y, $C_2H_4$-NH-$C_2H_4$-$SO_2$-Y, $C_2H_4$-$N(CH_3)$-$C_2H_4$-$SO_2$-Y, $C_2H_4OC_2H_4OC_2H_4$-$SO_2$-Y, $C_2H_4$-NH-$C_2H_4$-NH-$C_2H_4$-$SO_2$-Y oder $C_2H_4$-$N(CH_3)$-$C_2H_4$-$N(CH_3)$-$C_2H_4$-$SO_2$-Y.

Reste Z sind z.B. $CH_2$, $(CH_2)_2$, $(CH_2)_3$, $(CH_2)_4$, $(CH_2)_5$, $(CH_2)_6$, $CH(CH_3)CH_2$, $CH(CH_3)CH(CH_3)$, $C_2H_4$-O-$C_2H_4$, $C_2H_4$-NH-$C_2H_4$, $C_2H_4$-$N(CH_3)$-$C_2H_4$, $C_2H_4$-O-$C_2H_4$-O-$C_2H_4$, $C_2H_4$-NH-$C_2H_4$-NH-$C_2H_4$, $C_2H_4$-$N(CH_3)$ -$C_2H_4$-$N(CH_3)$ -$C_2H_4$ oder $C_2H_4$-O-$C_2H_4$-$N(CH_3)$ -$C_2H_4$.

Reste $R^3$ sind, wie weiterhin auch Reste $L^2$, Phenyl, 2-, 3- oder 4-Hydroxysulfonylphenyl, 2-, 3- oder 4-Chlorphenyl, 2-, 3- oder 4-Nitrophenyl, 2-, 3- oder 4-Methylphenyl, 2-, 3- oder 4-Methoxyphenyl, 2-, 3- oder 4-Cyanophenyl,

2-, 3- oder 4-Carboxylphenyl, 2-, 3- oder 4-Acetylaminophenyl, 2-, 3- oder 4-Hydroxysulfonylmethylphenyl oder Reste der Formel

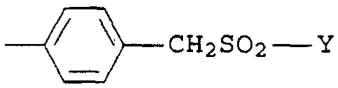, 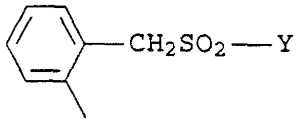, 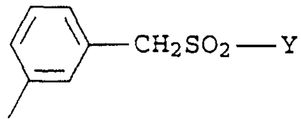,

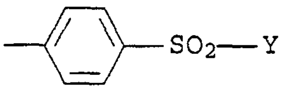, 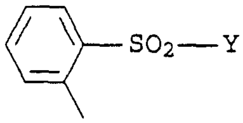, 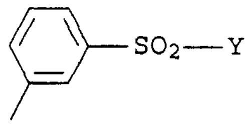,

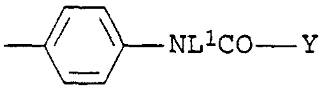, 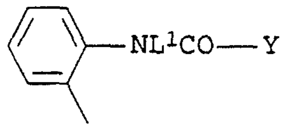, 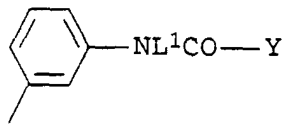,

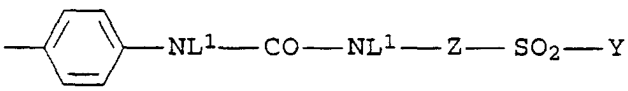,

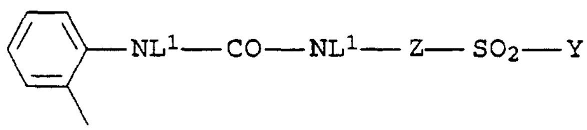,

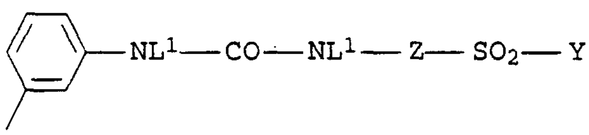,

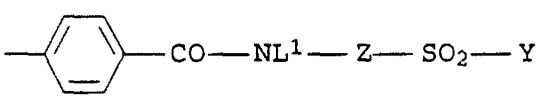,

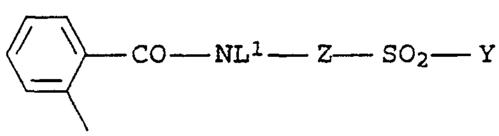 oder

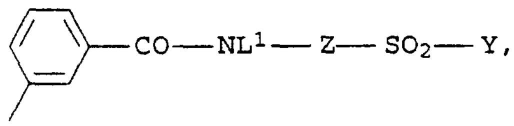

Wenn in Formel I der Rest $NL^1L^2$ mehrfach auftritt, so können die Reste voneinander verschieden sein. Gleiches gilt für $OL^1$. Bevorzugt werden Verbindungen der Formel I, in denen die Reste $NL^1L^2$ bzw. $OL^1$ gleich sind.
Ferner sind mehrere Reste $L^1$ beispielsweise im Rest $R^3$ voneinander unabhängig.
Bevorzugt sind Triazinderivate der Formel I, in der $R^1$, $R^2$ und $R^3$ voneinander unabhängige Reste der Formel $NL^1L^2$ bedeuten.
Bevorzugt sind weiterhin Triazinderivate der Formel I, in der $L^1$ und $L^2$ unabhängig voneinander jeweils Wasserstoff, $C_1$-$C_4$-Alkyl oder $C_1$-$C_{10}$-Alkyl, das mit einem Rest der Formel $-SO_2$-Y substituiert ist und gegebenenfalls durch jeweils 1 bis 3 Sauerstoffatome in Etherfunktion oder nicht benachbarte Imino- oder $C_1$-$C_4$-Alkyliminogruppen unterbrochen ist, bedeuten.
Bevorzugt sind weiterhin Triazinderivate der Formel I, in der Y Vinyl, 2-Sulfatoethyl, 2-Chlorethyl oder 2-Acetyloxyethyl bedeutet.
Besonders bevorzugt sind Triazinderivate der Formel I, in der $L^1$ und $L^2$ unabhängig voneinander jeweils Wasserstoff oder $C_1$-$C_8$-Alkyl, das mit einem Rest der Formel $-SO_2$-Y substituiert ist und gegebenenfalls durch jeweils 1 oder 2 Sauerstoffatome in Etherfunktion oder nicht benachbarte Imino- oder $C_1$-$C_4$-Alkyliminogruppen unterbrochen ist, bedeuten.
Ganz besonders bevorzugt sind Triazinderivate der Formel I, in der $L^1$ Wasserstoff oder $C_1$-$C_6$-Alkyl, das mit einem Rest der Formel $-SO_2$-Y substituiert ist und gegebenenfalls durch ein Sauerstoffatom in Etherfunktion oder eine Imino- oder $C_1$-$C_4$-Alkyliminogruppe unterbrochen ist, und $L^2$ Wasserstoff bedeuten.
Von besonderem Interesse sind Triazinderivate der Formel Ia

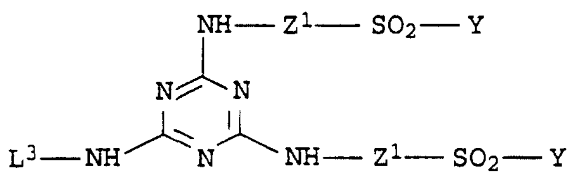

(Ia),

in der

- $Z^1$ $C_2$-$C_6$-Alkylen, das gegebenenfalls durch ein Sauerstoffatom in Etherfunktion unterbrochen ist, und
- $L^3$ Wasserstoff oder den Rest $Z^1$-$SO_2$-Y bedeuten und
- Y die obengenannte Bedeutung besitzt.

Die neuen Triazinderivate der Formel I können auf an sich bekannte Weise erhalten werden. Beispielsweise kann man Cyanurhalogenide der Formel II

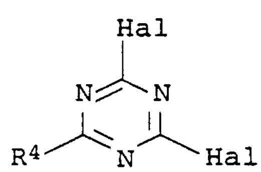

(II),

in der Hal Halogen, insbesondere Chlor, und $R^4$ Halogen, insbesondere Chlor, $C_1$-$C_4$-Alkyl oder Phenyl, das ein- oder zweifach durch Hydroxysulfonyl, Chlor, Nitro, $C_1$-$C_4$-Alkyl, $C_1$-$C_4$-Alkoxy, Cyano, Carboxyl, Acetylamino, Hydroxysulfonylmethyl oder einen Rest der Formel $CH_2$-$SO_2$-Y, $SO_2$-Y, $NL^1$-CO-Y oder $NL^1$-CO-$NL^1$-Z-$SO_2$-Y substituiert sein kann, bedeuten, mit Aminen der Formel III und/ oder Hydroxyverbindungen der Formel IV

$$NHL^1L^2 \qquad (III)$$

$$L^1OH \qquad (IV),$$

worin $L^1$ und $L^2$ jeweils die obengenannte Bedeutung besitzen, umsetzen.

Eine andere Möglichkeit besteht darin, Aminotriazine der Formel V

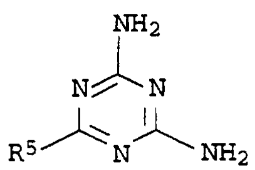

(V),

in der $R^5$ Amino, $C_1$-$C_4$-Alkyl oder Phenyl, das ein- oder zweifach durch Hydroxysulfonyl, Chlor, Nitro, $C_1$-$C_4$-Alkyl, $C_1$-$C_4$-Alkoxy, Cyano, Carboxyl, Acetylamino, Hydroxysulfonylmethyl oder einen Rest der Formel $CH_2$-$SO_2$-Y, $SO_2$-Y, $NL^1$-CO-Y oder $NL^1$-CO-$NL^1$-Z-$SO_2$-Y substituiert sein kann, bedeutet, unter Ammoniakabspaltung mit Aminen der Formel III und/oder Hydroxyverbindungen der Formel IV umzusetzen.

Die erfindungsgemäßen Triazinderivate eignen sich in vorteilhafter Weise als Fixierhilfsmittel beim Färben von Hydroxygruppen oder Stickstoffatome aufweisenden organischen Substraten mittels anionischer Farbstoffe.

Geeignete Substrate sind beispielsweise Leder oder Fasermaterial, das überwiegend natürliche oder synthetische Polyamide oder natürliche oder regenerierte Cellulose enthält. Weiterhin geeignet sind keratinische Fasern wie Haare und Pelze. Vorzugsweise handelt es sich um Textilmaterial auf der Basis von Wolle oder insbesondere von Baumwolle.

Geeignete anionische Farbstoffe sind z.B. solche, die eine oder mehrere Hydroxysulfonyl-, Aminosulfonyl- und/oder Carboxylgruppe aufweisen. Zusätzlich können sie auch noch eine oder mehrere faserreaktive Gruppen aufweisen.

Sie stammen z.B. aus der Klasse der Mono- oder Polyazofarbstoffe, der metallisierten Formazanfarbstoffe, beispielsweise der Kupfer-Formazanfarbstoffe, der Anthrachinonfarbstoffe, der Triphendioxazinfarbstoffe oder der Phthalocyaninfarbstoffe.

Anstelle der Azofarbstoffe können auch die entsprechenden Metallkomplexazofarbstoffe Anwendung finden. Als komplexierende Metalle kommen dabei insbesondere Kupfer, Kobalt, Chrom, Nickel oder Eisen in Betracht, wobei Kupfer, Kobalt oder Chrom bevorzugt sind.

Dabei befinden sich die metallisierten Gruppen vorzugsweise jeweils in ortho-Stellung zur Azogruppe, z.B. in Form von o,o'-Dihydroxy-, o-Hydroxy-o'-carboxyl-, o-Carboxyl-o'-aminooder o-Hydroxy-o'-amino-azogruppierungen.

Die genannten Farbstoffe sind allgemein bekannt und handelsüblich und beispielsweise im Colour Index unter den Namen "Acid Dyes" oder "Reactive Dyes" beschrieben.

Die Wirkungsweise der Fixierhilfsmittel ist bekannt und z.B. in K. Venkataraman (loc. cit.) beschrieben. Dabei werden Farbstoffe, die eine nucleophile Gruppe aufweisen mittels der Fixierhilfsmittel an die Hydroxygruppen oder Stickstoffatome aufweisenden organischen Substrate gebunden.

Eine besondere Möglichkeit der Anwendung der erfindungsgemäßen Triazinderivate als Fixierhilfsmittel besteht z.B. darin, sie direkt in fertigen Präparationen zusammen mit dem anionischen Farbstoff anzuwenden. Diese Präparationen können z.B. Pulver oder Granulate sein, die durch gemeinsame Sprühtrocknung der Syntheselösungen von Triazinderivat und anionischem Farbstoff erhalten werden können.

Weiterhin eignen sich die erfindungsgemäßen Triazinderivate als Vernetzer für Regeneratcellulose oder cellulosehaltigen Materialien, wie beispielsweise in der EP-A 538 977 beschrieben.

Solche Triazinderivate der Formel I, die über mindestens zwei faserreaktive Gruppen im Molekül verfügen, sind auch als Anker zur Herstellung von Reaktivfarbstoffen geeignet.

Die folgenden Beispiele sollen die Erfindung näher erläutern.

Beispiel 1

a) Zu einem Gemisch aus 229 g Melamin und 1530 g 2,2'-Aminoethoxyethanol wurden unter Rühren 37,5 g konz. Schwefelsäure getropft. Anschließend wurde unter Rühren und Überleiten eines schwachen Stickstoffstroms 11 h auf 210 bis 220°C erhitzt.

Nach Abkühlen wurde mit 58,3 g 50 gew.-%iger Natronlauge versetzt und vom ausgefallenen Natriumsulfat abfiltriert. Anschließend wurde überschüssiges 2,2'-Aminoethoxyethanol unter vermindertem Druck abdestilliert. Man erhielt 702 g N,N',N''-Tris(5-hydroxy-3-oxapentyl)melamin als gelbliches Harz, Reinheit: 96 bis 98 % (HPLC).

b) 296 g der unter a) beschriebenen Verbindung trug man bei Raumtemperatur in 600 g Thionylchlorid ein. Nach beendeter exothermer Reaktion erhitzte man weitere 3 h auf 60 bis 70°C. Man ließ abkühlen und fällte durch Zugabe von 2000 g Petrolether. Nach Absaugen und Trocknen isolierte man 243 g N,N',N''-Tris(5-chlor-3-oxapentyl)melamin.

c) 270 g 30 gew.-%ige methanolische Natriummethanolatlösung und 94 g 2-Mercaptoethanol wurden in 300 g n-Butanol auf 115 bis 118°C erhitzt. Nachdem das Abdestillieren von Methanol beendet war, gab man 135 g der unter b) beschriebenen Verbindung zu und erhitzte 3 h unter Rückfluß. Man ließ abkühlen, gab 1000 ml Wasser zu und trennte die organische Phase ab. Nach Abdestillieren des n-Butanols isolierte man 135 g der Verbindung der Formel

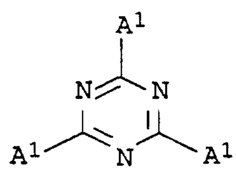

($A^1$ $= NHC_2H_4OC_2H_4SC_2H_4OH$)

als leicht braunes Öl.

d) Zu einer Mischung aus 45,6 g der unter c) beschriebenen Verbindung, 3,5 g Essigsäure, 0,5 g Wolframsäure und 150 ml Wasser, tropfte man bei 60°C unter stark exothermer Reaktion 57 g 30 gew.-%iges Wasserstoffperoxid. Nach 3 h bei 85°C war kein Peroxid mehr nachweisbar. Man destillierte das Lösungsmittel unter vermindertem Druck ab und isolierte 55 g der Verbindung der Formel

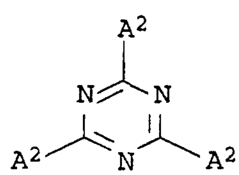

($A^2$ $= NHC_2H_4OC_2H_4SO_2C_2H_4OH$)

als bräunliches Harz.

e) 107 g der unter d) beschriebenen Verbindung trug man so langsam in 500 g reine Schwefelsäure ein, daß die Temperatur 25°C nicht überstieg. Nach 10 h goß man die homogene Lösung auf 3000 g Eis, stellte den pH-Wert mit festen Calciumcarbonat auf 4,5 und saugte ab. Das Filtrat wurde unter vermindertem Druck bei einer Temperatur < 30°C eingeengt. Als Rückstand verblieben 165 g eines braunen Harzes, das im Massenspektrum (Elektrospray Ionisation) einen Molpeak bei 906 zeigt und der Formel

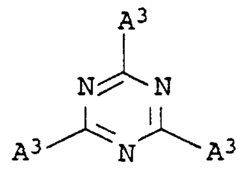

$(A^3 = NHC_2H_4OC_2H_4SO_2C_2H_4OSO_3H)$

entspricht.

Beispiel 2

a) Zu 46 g Cyanurchlorid und 69 g wasserfreiem Kaliumcarbonat in 1000 g Dioxan wurden 97 g 2-Hydroxyethyl-2'-aminoethylsulfid getropft. Das Reaktionsgemisch wurde 7 h unter Überleiten eines schwachen Stickstoffstroms zum Rückfluß erhitzt.

Nach Abkühlen wurde abfiltriert und das Filtrat eingedampft. Das erhaltene gelbe Öl wurde zur Reinigung mit Isopropanol an Kieselgel chromatographiert. Als erste Fraktion eluierte man N,N',N"-Tris(5-hydroxy-3-thiapentyl) melamin. Ausbeute: 88 g gelbliches Harz.

b) Zu einer Mischung aus 294 g der unter a) beschriebenen Verbindung, 50 g Essigsäure, 2 g Wolframsäure und 600 ml Wasser, tropfte man bei 80 bis 85°C 453 g 30 gew.-%iges Wasserstoffperoxid. Nach weiteren 2 h bei 80 bis 85°C wurde filtriert und das Filtrat unter vermindertem Druck eingeengt Man isolierte 350 g der Verbindung der Formel

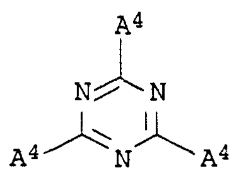

$(A^4 = NHC_2H_4SO_2C_2H_4OH)$

c) 81 g der unter b) beschriebenen Verbindung trug man bei einer Temperatur < 25°C in 700 g 5 gew.-%iges Oleum ein. Nach 10 h wurde auf 3500 g Eis gegossen und der pH-Wert durch Zugabe von Calciumcarbonat auf 5,5 gestellt. Man saugte ab und gab die Mutterlauge zur Sprühtrocknung. Man isolierte 70 g eines leicht gelblichen, gut wasserlöslichen Pulvers (Fp. < 300°C), das nach Massenspektrum (Elektrospray Ionisation) der Formel

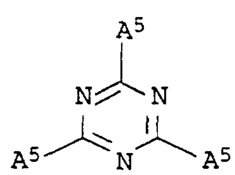

$(A^5 = NHC_2H_4SO_2C_2H_4OSO_3H)$

entspricht.

Beispiel 3 (Anwendung)

Baumwollgewebe wird auf einem Zweiwalzenfoulard bei Raumtemperatur mit einer Flotte imprägniert, die in

1000 Gewichtsteilen Wasser 40 Gewichtsteile des Farbstoffs der Formel

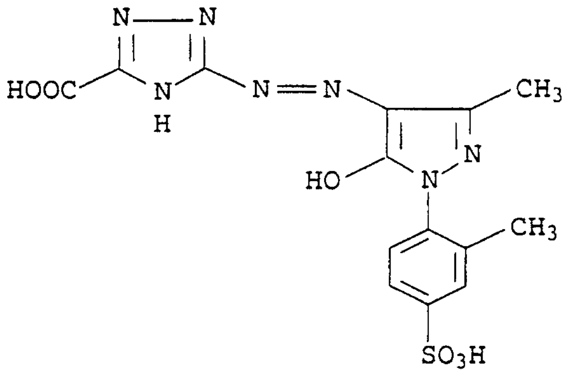

,

40 Gewichtsteile des in Beispiel 2c beschriebenen Triazinderivats und 20 Gewichtsteile 32 gew.-%ige Natronlauge enthält. Nach dem Abquetschen mit einer Flottenaufnahme von 70 % wird bei 80°C zwischengetrocknet und dann das Gewebe während 8 min bei 102°C gedämpft. Nach dem Spülen und kochenden Seifen erhält man eine tiefe grünstichige Gelbfärbung mit hervorragenden Fabrikationsund Gebrauchsechtheiten, insbesondere mit sehr guter Licht- und Waschechtheit.

Beispiel 4 (Anwendung)

Baumwollgewebe wird auf einem Zweiwalzenfoulard bei Raumtemperatur mit einer Flotte imprägniert, die in 1000 Gewichtsteilen Wasser 80 Gewichtsteile des Farbstoffs der Formel

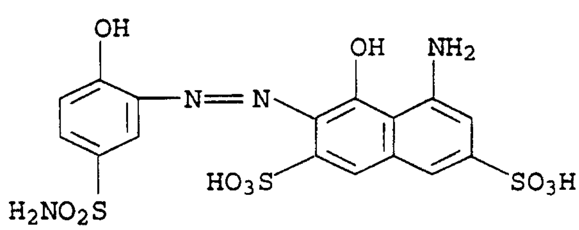

,

(1:2-Co-Komplex)

40 Gewichtsteile des in Beispiel 2c beschriebenen Triazinderivats, 20 Gewichtsteile 32 gew.-%ige Natronlauge und 50 Gewichtsteile Natronwasserglas (38°Bé) enthält. Nach dem Abquetschen mit einer Flottenaufnahme von 70 % wird das Gewebe aufgerollt und nach Umschließen mit einer Folie für 24 h bei Raumtemperatur in feuchtem Zustand gelagert. Nach dem Spülen und kochenden Seifen erhält man eine tiefe Graufärbung mit hervorragenden Fabrikations- und Gebrauchsechtheiten.

Beispiel 5 (Anwendung)

100 g Pelz werden in einem Bad, das in 5000 Gewichtsteilen Wasser 5 Gewichtsteile des Farbstoffs der Formel

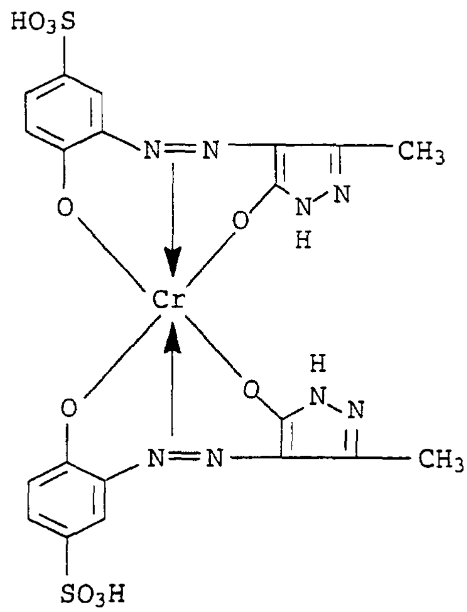

und 5 Gewichtsteile des in Beispiel 2c beschriebenen Triazinderivats enthält bei einem pH-Wert von 3,5 (eingestellt mit 10 gew.-%iger Essigsäure) gefärbt.

Man beginnt mit dem Färben bei Raumtemperatur, erhöht innerhalb von 20 Minuten auf 60°C und färbt bei dieser Temperatur weitere 40 Minuten. Dann wird der pH-Wert des Färbebades durch Zugabe einer 25 Gew.-%igen Ammoniaklösung auf pH 8,0 eingestellt und bei diesem pH-Wert weitere 20 Minuten bei 60°C gefärbt.

Nach dem Spülen, zunächst in 50°C warmen und dann in kaltem Wasser, erhält man eine orangefarbene Pelzfärbung mit guten Echtheiten.

## Patentansprüche

1. Triazinderivate der Formel I

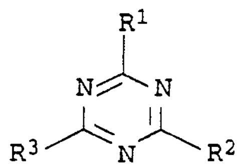

(I),

in der die Variablen die folgende Bedeutung haben:

$R^1$ ist ein Rest der Formel $NL^1L^2$ oder $OL^1$;

$R^2$ ist ein Rest der Formel $NL^1L^2$ oder $OL^1$ oder Halogen;

$R^3$ ist ein Rest der Formel $NL^1L^2$ oder $OL^1$, $C_1$-$C_4$-Alkyl oder Phenyl, das ein- oder zweifach durch Hydroxysulfonyl, Chlor, Nitro, $C_1$-$C_4$-Alkyl, $C_1$-$C_4$-Alkoxy, Cyano, Carboxyl, Acetylamino, Hydroxysulfonylmethyl oder einen Rest der Formel

$$CH_2\text{-}SO_2\text{-}Y,\ SO_2\text{-}Y,\ NL^1\text{-}CO\text{-}Y \text{ oder } NL^1\text{-}CO\text{-}NL^1\text{-}Z\text{-}SO_2\text{-}Y$$

substituiert sein kann;

$L^1$ ist Wasserstoff oder $C_1$-$C_{10}$-Alkyl, das mit einem Rest der Formel $-SO_2-Y$ substituiert sein kann und gegebenenfalls durch jeweils 1 bis 3 Sauerstoffatome in Etherfunktion oder nicht benachbarte Imino- oder $C_1$-$C_4$-Alkyliminogruppen unterbrochen ist;

$L^2$ ist ein Rest $L^1$ oder Phenyl, das ein- oder zweifach mit Hydroxysulfonyl, Chlor, Nitro, $C_1$-$C_4$-Alkyl, $C_1$-$C_4$-Alkoxy, Cyano, Carboxyl, Acetylamino, Hydroxysulfonylmethyl oder einem Rest der Formel

$$CH_2-SO_2-Y,\ SO_2-Y,\ NL^1-CO-Y,\ CO-NL^1-Z-SO_2-Y \text{ oder}$$

$$NL^1-CO-NL^1-Z-SO_2-Y$$

substituiert sein kann,
oder $L^1$ und $L^2$ sind, zusammen mit dem sie verbindenden Stickstoffatom, Pyrrolidinyl, Piperidinyl, Morpholinyl, Piperazinyl oder N-($C_1$-$C_4$-Alkyl)piperazinyl;

Z ist $C_2$-$C_6$-Alkylen, das gegebenenfalls mit Hydroxy, Chlor, Cyano, Carboxyl, $C_1$-$C_4$-Alkoxycarbonyl, $C_1$-$C_4$-Alkanoyloxy oder Sulfato substituiert ist und durch jeweils 1 oder 2 Sauerstoffatome in Etherfunktion oder nicht benachbarte Imino- oder $C_1$-$C_4$-Alkyliminogruppen unterbrochen sein kann;

Y ist Vinyl oder ein Rest der Formel $C_2H_4$-Q, worin Q Chlor, Brom, $C_1$-$C_4$-Alkylsulfonyl, Phenylsulfonyl, $OSO_3H$, $SSO_3H$, $OP(O)(OH)_2$, $C_1$-$C_4$-Alkylsulfonyloxy, Phenylsulfonyloxy, $C_1$-$C_4$-Alkanoyloxy, $C_1$-$C_4$-Dialkylamino oder ein Rest der Formel

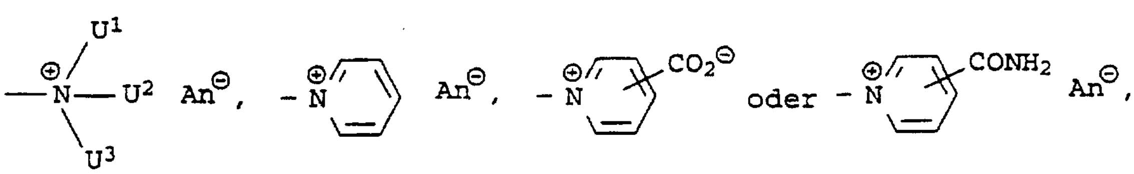

bedeutet,
wobei $U^1$, $U^2$ und $U^3$ unabhängig voneinander jeweils die Bedeutung von $C_1$-$C_4$-Alkyl oder Benzyl und $An^{\ominus}$ jeweils Fluorid, Chlorid, Bromid, Iodid, Mono-, Di- oder Trichloracetat, Methansulfonat, Benzolsulfonat oder 2- oder 4-Methylbenzolbedeutet,

mit der Maßgabe, daß im Rest $R^1$ $L^1$ $C_1$-$C_{10}$-Alkyl, das mit einem Rest der Formel $-SO_2-Y$ substituiert ist und gegebenenfalls durch jeweils 1 bis 3 Sauerstoffatome in Etherfunktion oder nicht benachbarte Imino- oder $C_1$-$C_4$-Alkyliminogruppen unterbrochen ist, bedeutet.

2. Triazinderivate nach Anspruch 1, **dadurch gekennzeichnet, daß** $R^1$, $R^2$ und $R^3$ voneinander unabhängige Reste der Formel $NL^1L^2$ bedeuten.

3. Triazinderivate nach Anspruch 1, **dadurch gekennzeichnet, daß** $L^1$ und $L^2$ unabhängig voneinander jeweils Wasserstoff, $C_1$-$C_4$-Alkyl oder $C_1$-$C_{10}$-Alkyl, das mit einem Rest der Formel $-SO_2-Y$ substituiert ist und gegebenenfalls durch jeweils 1 bis 3 Sauerstoffatome in Etherfunktion oder nicht benachbarte Imino- oder $C_1$-$C_4$-Alkyliminogruppen unterbrochen ist, bedeuten.

4. Triazinderivate nach Anspruch 1, **dadurch gekennzeichnet, daß** $L^1$ und $L^2$ unabhängig voneinander jeweils Wasserstoff oder $C_1$-$C_8$-Alkyl, das mit einem Rest der Formel $-SO_2-Y$ substituiert ist und gegebenenfalls durch jeweils 1 oder 2 Sauerstoffatome in Etherfunktion oder nicht benachbarte Imino- oder $C_1$-$C_4$-Akyliminogruppen unterbrochen ist, bedeuten.

5. Triazinderivate nach Anspruch 1, **dadurch gekennzeichnet, daß** $L^1$ Wasserstoff oder $C_1$-$C_6$-Alkyl, das mit einem

Rest der Formel $-SO_2-Y$ substituiert ist und gegebenenfalls durch ein Sauerstoffatom in Etherfunktion oder eine Imino- oder $C_1$-$C_4$-Akyliminogruppe unterbrochen ist, und $L^2$ Wasserstoff bedeuten.

**6.** Verwendung der Triazinderivate gemäß Anspruch 1 als Fixierhilfsmittel beim Färben von Hydroxygruppen oder Stickstoffatome aufweisenden organischen Substraten mittels anionischer Farbstoffe.

**7.** Verwendung der Triazinderivate gemäß Anspruch 1 als Vernetzer für Regeneratcellulose oder cellulosehaltige Materialien.

## Claims

**1.** A triazine derivative of the formula I

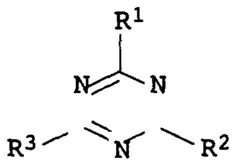

(I),

where

$R^1$ is $NL^1L^2$ or $OL^1$;

$R^2$ is $NL^1L^2$ or $OL^1$ or halogen;

$R^3$ is $NL^1L^2$ or $OL^1$, $C_1$-$C_4$-alkyl or phenyl, which may be substituted once or twice by hydroxysulfonyl, chlorine, nitro, $C_1$-$C_4$-alkyl, $C_1$-$C_4$-alkoxy, cyano, carboxyl, acetylamino, hydroxysulfonylmethyl or

$$CH_2—SO_2—Y,\ SO_2—Y,\ NL^1—CO—Y \text{ or}$$

$$NL^1—CO—NL^1—Z—SO_2—Y\ ;$$

$L^1$ is hydrogen or $C_1$-$C_{10}$-alkyl which can be substituted by $-SO_2-Y$ and which is uninterrupted or interrupted each time by 1-3 oxygens in ether function or by nonadjacent iminos or $C_1$-$C_4$-akyliminos;

$L^2$ is $L^1$ or phenyl which can be substituted once or twice by hydroxysulfonyl, chlorine, nitro, $C_1$-$C_4$-alkyl, $C_1$-$C_4$-alkoxy, cyano, carboxyl, acetylamino, hydroxysulfonylmethyl or

$$CH_2—SO_2—Y,\ SO_2—Y,\ NL^1—CO—Y,$$

$$CO—NL^1—Z—SO_2—Y \text{ or}$$

$$NL^1—CO—NL^1—Z—SO_2—Y,$$

or $L^1$ and $L^2$, together with the nitrogen linking them, are pyrrolidinyl, piperidinyl, morpholinyl, piperazinyl or N-($C_1$-$C_4$-alkyl)piperazinyl;

Z is $C_2$-$C_6$-alkylene which is unsubstituted or substituted by hydroxyl, chlorine, cyano, carboxyl, $C_1$-$C_4$-alkoxycarbonyl, $C_1$-$C_4$-alkanoyloxy or sulfato and may be interrupted each time by 1 or 2 oxygens in ether function or by nonadjacent iminos or $C_1$-$C_4$-alkyliminos;

Y is vinyl or $C_2H_4$-Q in which Q is chloro, bromo, $C_1$-$C_4$-alkylsulfonyl, phenylsulfonyl, $OSO_3H$, $SSO_3H$, $OP(O)(OH)_2$, $C_1$-$C_4$-alkylsulfonyloxy, phenylsulfonyloxy, $C_1$-$C_4$-alkanoyloxy, $C_1$-$C_4$-dialkylamino or

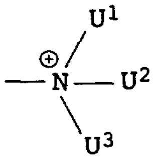 $An^{\ominus}$, 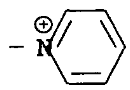 $An^{\ominus}$, 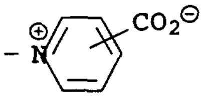 or 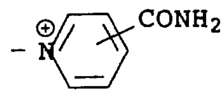 $An^{\ominus}$,

where $U^1$, $U^2$ and $U^3$ independently are each $C_1$-$C_4$-alkyl or benzyl and And is fluoride, chloride, bromide, iodide and mono-, di- or trichloroacetate, methanesulfonate, benzenesulfonate and 2- or 4-methylbenzenesulfonate,
with the proviso that in $R^1$ $L^1$ is $C_1$-$C_{10}$-alkyl substituted by $-SO_2$-Y and is uninterrupted or interrupted each time by 1-3 oxygens in ether function or by nonadjacent iminos or $C_1$-$C_4$-alkyliminos.

**2.** A triazine derivative as claimed in claim 1, wherein $R^1$, $R^2$ and $R^3$ independently are radicals of the formula $NL^1L^2$.

**3.** A triazine derivative as claimed in claim 1, wherein $L^1$ and $L^2$ independently are each hydrogen, $C_1$-$C_4$-alkyl or $C_1$-$C_{10}$-alkyl which is substituted by $-SO_2$-Y and is uninterrupted or interrupted each time by 1 - 3 oxygens in ether function or by nonadjacent iminos or $C_1$-$C_4$-alkyliminos.

**4.** A triazine derivative as claimed in claim 1, wherein $L^1$ and $L^2$ independently are each hydrogen or $C_1$-$C_8$-alkyl which is substituted by $-SO_2$-Y and is uninterrupted or interrupted each time by 1 or 2 oxygens in ether function or by nonadjacent iminos or $C_1$-$C_4$-alkyliminos.

**5.** A triazine derivative as claimed in claim 1, wherein $L^1$ is hydrogen or $C_1$-$C_6$-alkyl which is substituted by $SO_2$-Y and is uninterrupted or interrupted by an oxygen in ether function or by an imino or $C_1$-$C_4$-alkylimino, and $L^2$ is hydrogen.

**6.** The use of a triazine derivative as claimed in claim 1 as a fixation aid when using anionic dyes to dye hydroxyl- or nitrogen-containing organic substrates.

**7.** The use of a triazine derivative as claimed in claim 1 as a crosslinker for regenerated cellulose or materials containing cellose.

**Revendications**

**1.** Dérivés de triazine de formule I

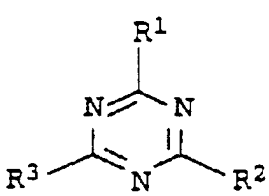

(I),

dans laquelle les variables ont les significations suivantes :

$R^1$ est un reste de formule $NL^1L^2$ ou $OL^1$ ;
$R^2$ est un reste de formule $NL^1L^2$ ou $OL^1$ ou un atome d'halogène ;
$R^3$ est un reste de formule $NL^1L^2$ ou $OL^1$, un groupe alkyle en $C_1$-$C_4$ ou phényle qui peut être une ou deux fois substitué par un ou des atomes de chlore ou groupes hydroxysulfonyle, nitro, alkyle en $C_1$-$C_4$, alcoxy en $C_1$-$C_4$,, cyano, carboxy, acétylamino, hydroxysulfonylméthyle ou par un radical de formule

$CH_2$-$SO_2$-Y, $SO_2$-Y, $NL^1$-CO-Y ou $NL^1$-CO-$NL^1$-Z-$SO_2$-Y ;

$L^1$ est un atome d'hydrogène ou un groupe alkyle en $C_1$-$C_{10}$ qui peut être substitué par un radical de formule $-SO_2-Y$ et est éventuellement interrompu par, respectivement, 1 à 3 atomes d'oxygène en fonction éther ou groupes imino ou alkyl($C_1$-$C_4$)imino non contigus ;

$L^2$ est un reste $L^1$ ou phényle qui peut être une ou deux fois substitué par un ou des atomes de chlore ou groupes hydroxysulfonyle, nitro, alkyle en $C_1$-$C_4$, alcoxy en $C_1$-$C_4$, cyano, carboxy, acétylamino, hydroxysulfonylméthyle ou par un radical de formule

$$CH_2\text{-}SO_2\text{-}Y,\ SO_2\text{-}Y,\ NL^1\text{-}CO\text{-}Y,\ CO\text{-}NL^1\text{-}Z\text{-}SO_2\text{-}Y \text{ ou } NL^1\text{-}CO\text{-}NL^1\text{-}Z\text{-}SO_2\text{-}Y\ ;$$

ou $L^1$ et $L^2$ forment ensemble, avec l'atome d'azote qui les relie, un groupe pyrrolidinyle, pipéridinyle, morpholinyle, pipérazinyle ou N-[alkyl($C_1$-$C_4$)]pipérazinyle ;

Z est un groupe alkylène en $C_2$-$C_6$ qui est éventuellement substitué par un atome de chlore ou par un groupe hydroxy, cyano, carboxy, alcoxy($C_1$-$C_4$)carbonyle, alcanoyloxy en $C_1$-$C_4$ ou sulfato et peut être interrompu par, respectivement, 1 à 2 atomes d'oxygène en fonction éther ou groupes imino ou alkyl($C_1$-$C_4$)imino non contigus ;

Y est un groupe vinyle ou un radical de formule $C_2H_4$-Q, dans laquelle Q représente un atome de chlore ou de brome ou un groupe alkyl($C_1$-$C_4$)sulfonyle, phénylsulfonyle, $OSO_3H$, $SSO_3H$, $OP(O)(OH)_2$, alkyl($C_1$-$C_4$)sulfonyloxy, phénylsulfonyloxy, alcanoyloxy en $C_1$-$C_4$, dialkyl($C_1$-$C_4$)amino ou un radical de formule

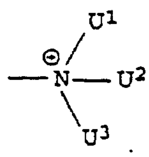 $An^\ominus$, 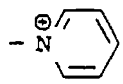 $An^\ominus$, 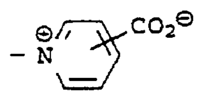 ou 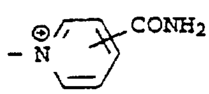 $An^\ominus$,

$U^1$, $U^2$ et $U^3$ représentant chacun, indépendamment les uns des autres, un groupe alkyle en $C_1$-$C_4$ ou benzyle et $An^-$ représentant dans chaque cas l'ion fluorure, chlorure, bromure, iodure, mono-, di- ou trichloracétate, méthanesulfonate, benzènesulfonate ou 2- ou 4-méthylbenzène, étant entendu que dans le reste $R^1$ $L^1$ représente un groupe alkyle en $C_1$-$C_{10}$ qui est substitué par un radical de formule $-SO_2-Y$ et est éventuellement interrompu par, respectivement, 1 à 3 atomes d'oxygène en fonction éther ou groupes imino ou alkyl($C_1$-$C_4$)imino non contigus.

2. Dérivés de triazine selon la revendication 1, **caractérisés en ce que** $R^1$, $R^2$ et $R^3$ représentent, indépendamment les uns des autres, des restes de formule $NL^1L^2$.

3. Dérivés de triazine selon la revendication 1, **caractérisés en ce que** $L^1$ et $L^2$ représentent chacun, indépendamment l'un de l'autre, un atome d'hydrogène, un groupe alkyle en $C_1$-$C_4$ ou alkyle en $C_1$-$C_{10}$ qui est substitué par un radical de formule $-SO_2-Y$ et est éventuellement interrompu par, respectivement, 1 à 3 atomes d'oxygène en fonction éther ou groupes imino ou alkyl($C_1$-$C_4$)imino non contigus.

4. Dérivés de triazine selon la revendication 1, **caractérisés en ce que** $L^1$ et $L^2$ représentent chacun, indépendamment l'un de l'autre, un atome d'hydrogène ou un groupe alkyle en $C_1$-$C_8$ qui est substitué par un radical de formule $-SO_2-Y$ et est éventuellement interrompu par, respectivement, 1 ou 2 atomes d'oxygène en fonction éther ou groupes imino ou alkyl($C_1$-$C_4$)imino non contigus.

5. Dérivés de triazine selon la revendication 1, **caractérisés en ce que** $L^1$ représente un atome d'hydrogène ou un groupe alkyle en $C_1$-$C_6$ qui est substitué par un radical de formule $-SO_2-Y$ et est éventuellement interrompu par un atome d'oxygène en fonction éther ou par un groupe imino ou alkyl($C_1$-$C_4$)imino, et $L^2$ représente un atome d'hydrogène.

6. Utilisation des dérivés de triazine selon la revendication 1, en tant qu'adjuvants de fixage dans la teinture de substrats organiques comportant des groupes hydroxy ou des atomes d'azote, au moyen de colorants anioniques.

7. Utilisation des dérivés de triazine selon la revendication 1, en tant qu'agent de réticulation pour cellulose régénérée ou matériaux contenant de la cellulose.